# EUROPEAN PATENT APPLICATION

(11) **EP 4 053 191 A1**
(43) Date of publication of application: **07.09.2022**
(21) Application number: 20887185.5
(22) Date of filing: 13.11.2020
(51) Int. Cl.: C08J 3/14, A61K 47/32, A61L 31/04, A61L 31/14, C12M 3/00, C12N 5/07

(54) **METHOD FOR HYDRATING WATER-INSOLUBLE POLYMER CAPABLE OF CONTAINING INTERMEDIATE WATER**

(30) Priority: 14.11.2019 JP 2019206564
(71) Applicant: Kyushu University, National University Corporation, Nishi-ku Fukuoka-shi Fukuoka 819-0395 (JP)
(72) Inventor: NISHIDA, Kei, Fukuoka-shi, Fukuoka 819-0395 (JP); UEHARA, Hiroki, Fukuoka-shi, Fukuoka 819-0395 (JP); TANAKA, Masaru, Fukuoka-shi, Fukuoka 819-0395 (JP)
(74) Representative: Hoffmann Eitle
(86) International application number: PCT/JP2020/042474
(87) International publication number: WO 2021/095862

(57) **Abstract**

A method for hydrating a water-insoluble polymer capable of containing intermediate water, comprising; a solution generation step of dissolving a water-insoluble polymer capable of containing intermediate water by hydration in a polar organic solvent to obtain a solution, and a precipitation step hydrating and precipitating the water-insoluble polymer by mixing the solution with an aqueous phase.

## Description

### Technical Field

The present invention relates to a method for hydrating a water-insoluble polymer capable of containing intermediate water, and a hydrate of the water-insoluble polymer obtained by the method. This application claims priority based on Japanese Patent Application No. 2019-206564 filed in Japan on November 14, 2019, the contents of which are incorporated herein by reference.

### Background Art

In general, it is known that when a biological component such as blood comes into contact with the surfaces of various materials, the surfaces of the materials are recognized as foreign matters and non-specific adsorption, denaturation, multi-layer adsorption and the like of proteins in biological tissues occur, resulting in activation of coagulation system, complement system, platelet system and the like. On the other hand, it is known that non-specific adsorption of the above-mentioned protein and the like is suppressed on a surface generated when a polymer having a specific structure is hydrated, and as a result, biocompatibility such as difficulty in causing platelet adhesion and the like is expressed.

It has been clarified that when the above-mentioned biocompatible polymers are hydrated, water molecules can be hydrated in a form commonly referred to as "freezing-bound water" (intermediate water). Intermediate water is characterized by the transfer of latent heat associated with the regularization/disordering of water molecules in the temperature range below zero. It is understood as a water molecule whose properties are intermediate between those of non-freezing water, which is strongly confined to a material surface, and free water, which is hardly confined by a material surface. It has been clarified that such intermediate water is observed in various bio-derived substances in addition to the above-mentioned polymers, and is considered to play an important role in the expression of biocompatibility (see, for example, Non-Patent Document 1).

Regarding the mechanism of generating water molecules in the form of intermediate water when a polymer having a specific structure as described above is hydrated, it has been clarified that the high molecular mobility exhibited by the polymer having the specific structure is related (see, for example, Non-Patent Document 2). In other words, it is considered that the high molecular mobility of the polymer when it is hydrated is the cause of the formation of water molecules in the form of intermediate water, and as a result, the biocompatibility is exhibited.

On the other hand, the present inventors have found that tumor cells such as metastatic cancer cells, stem cells, vascular endothelial cells and the like contained in blood and the like can selectively adhere to a surface to which blood components are difficult to adhere by containing intermediate water, and the cell types can be selectively separated from blood and the like (Patent Document 1). Further, it has been disclosed that a surface containing intermediate water at a predetermined ratio is suitable for culturing various cells (Patent Document 2).

By utilizing various phenomena occurring between a polymer holding intermediate water at a predetermined ratio as described above and various biological substances, the polymer is expected to be used in various applications other than imparting biocompatibility to the surface of a medical device.

### Citation List

### Patent Literature

Patent Literature 1: Japanese Unexamined Patent Publication No. 2012-105579
Patent Literature 2: Japanese Unexamined Patent Publication No. 2016-63801
Patent Literature 3: Japanese Unexamined Patent Publication No. H03-39309

### Non-Patent Literature

Non-Patent Literature 1: Koubunshi, Vol.63, No.8, (2014)
Non-Patent Literature 2: Kagaku, Vol.66, No.5, (2011)

### Summary of Invention

### Technical Problem

It is known that polymers which can contain intermediate water by hydration as described above are hydrophilic, and many of them are water soluble. For example, polyethylene glycol (PEG), polyvinyl pyrrolidone (PVP) and methacryloyl phosphatidylcholine (MPC), which are known as biocompatible polymers, are all water-soluble. For this reason, when a surface exhibiting biocompatibility is to be formed by PEG, MPC, or the like, it is generally used by fixing it in a film form on the surface of a base material by a method such as making it a copolymer with other water-insoluble components (see, for example, Patent Document 3).

On the other hand, it is known that there exists a polymer such as poly (2-methoxyethyl acrylate) (PMEA), which shows biocompatibility by being hydrated to contain intermediate water and shows non-water solubility. Such a water-insoluble biocompatible polymer can be used in the form of a homopolymer for the purpose of forming a biocompatible surface by dissolving it in an organic solvent or the like and coating and fixing it on the surfaces of various substrates in a film form.

However, for example, when a water-insoluble biocompatible polymer such as the above-mentioned PMEA is coated on the surface of a base material in a film form and fixed thereto, there is a trade-off relationship between being water-insoluble and containing intermediate water by hydration, such as requiring a long time for bringing water into contact with the PMEA film to saturate the film with water. A means for promoting hydration of such a water-insoluble biocompatible polymer is desired.

An object of the present invention is to provide a means for inducing hydration by a method different from the conventional method with respect to a water-insoluble polymer which can contain intermediate water by hydration, and to provide a water-insoluble polymer which is hydrated by the method.

### Solution to Problem

In order to solve the above problems, the present invention has the following features.
<1> A method for hydrating a water-insoluble polymer capable of containing intermediate water, which comprises a solution generation step in which a water-insoluble polymer capable of containing intermediate water is dissolved in a polar organic solvent by hydration to obtain a solution, and a precipitation step in which the water-insoluble polymer is hydrated and precipitated by mixing the solution with an aqueous phase.
<2> A method for hydrating a water-insoluble polymer capable of containing intermediate water, wherein the water-insoluble polymer is a polymer having at least a part of a side chain portion containing a chain ether structure or a cyclic ether structure with respect to the main chain skeleton.
<3> A method for hydrating a water-insoluble polymer capable of containing intermediate water, wherein the water-insoluble polymer is precipitated in a colloidal state in the precipitation step.
<4> The precipitation step is a method for hydrating a water-insoluble polymer capable of containing the intermediate water by mixing the solution and the aqueous phase through a semi-permeable membrane.
<5> Disclosed is a method for hydrating a water-insoluble polymer, which can further contain the aforementioned intermediate water in which a fat-soluble drug is dissolved in the aforementioned solution.
<6> A composition containing a hydrated water-insoluble polymer molecule which is obtained by mixing an aqueous phase with a solution obtained by dissolving a water-insoluble polymer capable of containing intermediate water by hydration in a polar organic solvent, thereby hydrating and precipitating the above-mentioned water-insoluble polymer.
<7> A composition comprising the aforementioned hydrated water-insoluble polymer molecules having a particle shape.
<8> A composition comprising the aforementioned hydrated water-insoluble polymer molecules having an average particle 100 µm or less.

### Advantageous Effects of Invention

By hydrating a water-insoluble polymer which can contain intermediate water by a method different from the conventional method, it is possible to rapidly hydrate the water-insoluble polymer and to make the water-insoluble polymer into fine particles.

### Brief Description of Drawings

FIG. 1 is a photograph showing a state of a mixed liquid obtained by mixing purified water and a PMEA solution.
FIG. 2 is a phase difference microscope image of a turbid white aggregate obtained by mixing purified water and a PMEA solution.
FIG. 3 is a phase difference microscope image of a turbid white aggregate obtained by mixing purified water and a polymer solution through a dialysis membrane.
FIG. 4 is a phase difference microscopic image of each cell after the white turbid aggregate is added to (a) HeLa cells and (b) NHDF cells and allowed to stand.
Figure 5A is a result of the hemolysis test indicated by the white turbid aggregate containing each polymer.
Figure 5B is survival rate of HeLa cells in the presence of turbid aggregates containing PMEA.
FIG. 6 is a result of observing a white turbid aggregate containing doxorubicin by a fluorescence microscope.
FIG. 7 is a diagram showing the results of ¹H NMR measurement of a white turbid aggregate and a deuterated chloroform solution of PMEA.
FIG. 8 is a graph showing changes in the number of molecules of H₂O and DMSO contained in the turbid white aggregate.
FIG. 9 shows a results of a cholesterol elution test from a cell.
Figure 10 shows correlation coefficients with fluorescent PMEA in lysosomes, endoplasmic reticulum, and mitochondria.
FIG. 11 shows phase difference images and fluorescence images of a cloud aggregates obtained by mixing each of fluoresceinated sugar chains.

### Description of Embodiments

(1) It is known that a polymer suitable for hydration by the method according to the present invention, for example, PMEA and its analogs, which is water-insoluble but can contain intermediate water by hydration, exists, and the polymer is dissolved in a specific polar solvent according to the structure of each polymer.

As described above, the present invention is characterized in that a polymer which is water-insoluble but can contain intermediate water by hydration is hydrated in a process of mixing the solution with an aqueous phase by using a solution in which the polymer is dissolved in a polar organic solvent in which the polymer is soluble.

In the hydration method according to the present invention, any polymer can be used without any particular limitation as long as it shows non-water-solubility when the synthesized bulk polymer is brought into contact with water, at the same time undergoes hydration with a predetermined amount of water molecules, and the presence of intermediate water in the polymer is confirmed by the hydration.

As a polymer suitable for hydration by the above-mentioned method according to the present invention, a polymer containing intermediate water when hydrated is preferably used because it contains a chain ether structure which is a structural unit mainly constituting PEG in a side chain portion thereof and a structure contributing to the inclusion of intermediate water, such as a cyclic ether structure, having an acrylic skeleton, a methacrylic skeleton, a polycarbonate skeleton, an alkylene skeleton, etc. as a main chain.

Examples of the above-mentioned polymers include those represented by the following formula (1) as those having a (meth)acrylic skeleton and a chain ether structure in the side chain portion. In the polymer represented by formula (1), it is known that a chain ether structure which is a chain alkyl oxide (CH₂-CH₂-O) which is a constituent unit of PEG is added to the main chain of the (meth) acrylic skeleton by an ester bond, and intermediate water can be contained in many structures. Examples of the polymer represented by formula (1) include poly(2-ethoxyethylacrylate), poly(2-methoxyethylacrylate), poly[2-(2-methoxyethoxy)ethyl methacrylate], poly[2-(2-ethoxyethoxy)ethyl acrylate], poly[2-(2-methoxyethoxy)ethoxy] ethyl methacrylate], poly[2-(2-methoxyethoxy)ethoxy]ethyl acrylate], poly[2-(2-methoxyethoxy)ethylacrylate], poly[2-(2-methoxyethoxy)ethyl methacrylate], poly[2-(2-ethoxyethoxy)ethyl methacrylate], poly[2-(2-ethoxyethoxy)ethyl methacrylate], PM2A (poly(2-(2-methoxyethoxy)ethyl acrylate-co-butyl acrylate)), PM3A (poly(2-(2-methoxyethoxy)ethyl acrylate-co-butyl acrylate)), poly(2-(2-ethoxyethoxyethyl)acrylate),poly(2-ethoxyethylvinylether), poly(tetrahydrofuran-3-ylmethylacrylate), poly[2-(methoxyethoxy)ethyl methacrylate], and the like. The polymer represented by formula (1) may be used as a polymer by polymerizing only a monomer having a single structure, or may be used as a copolymer containing a plurality of unit structures. 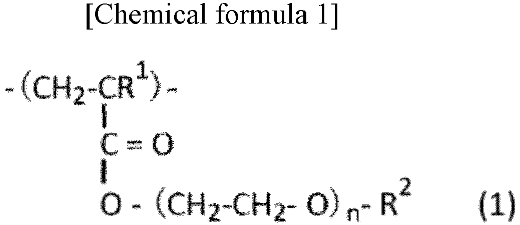 [wherein R¹ is an atom or a methyl group, R² is a methyl or an ethyl group, and n is 1 to 3]

Among the polymers represented by formula (1) above, poly (2-methoxyethyl acrylate (PMEA)) represented by formula (2) below and the like are particularly preferred because they have excellent biocompatibility and are already used for a plurality of bio-related applications. The above-mentioned PMe2A having n=2 as the repeating number (n in formula (1)) of ethylene glycol contained in the side chain of PMEA and the above-mentioned PMe3A having n = 3 as the repeating number are also preferably used because they can contain intermediate water at a large ratio.

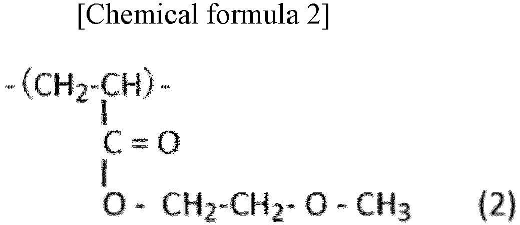

Examples of the above-mentioned polymer include those represented by the following formulae (3) and (4) as those having a vinyl skeleton and having a chain ether structure or a cyclic ether structure in a side chain portion. The polymers represented by formulae (3) and (4) have a repeating unit in which a side chain portion containing an ether structure is bonded to the main chain of the vinyl skeleton by an ether bond. The ether structure is a structure of a polyoxyalkylene group which may have a substituent, and as one form thereof, a chain ether or a cyclic ether which is a chain alkyl oxide can be mentioned.

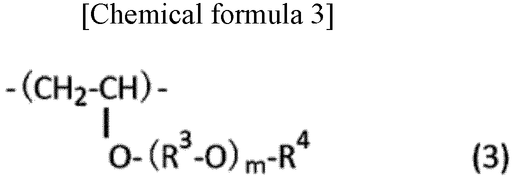

In general formula (3), R³ is a straight chain or branched alkyl group of CI to C4, and preferably R³ means any one of CH₂, C₂H₄, C₃H₆ and C₄H₈. R⁴ is H or a linear or branched alkyl group of C1 to C4, and preferably R⁴ means any one of H, CH₃, C₂H₅, C₃H₇ or C₄H₉. M is a natural number of 1 to 10, preferably within the range of 1 to 4, and more preferably 1 or 2. Here, the part (R³-O) represents a chain ether structure which is a unit structure such as PEG.

The polymer compound according to the present invention may contain a repeating unit represented by general formula (3) having mutually different R³, R⁴ and m values. That is, in the polymer compound according to the present invention, the organic component (R) of silsesquioxane may include a structure in which a main chain composed mainly of carbon is bonded to a monoether (m=1) having a terminal end terminated by a hydrogen or an alkyl group or a polyether (m≥2) by an ether bond so as to form a side chain. For example, when R³ is C₂H₄, the polymer compound of the present invention has a structure in which a chain ether (C₂H₄-O), which is a structural unit of PG terminated with an alkyl group or the like, is bonded to the main chain by an ether bond. Examples of the polymer represented by formula (3) include methoxyethyl vinyl ether and the like.

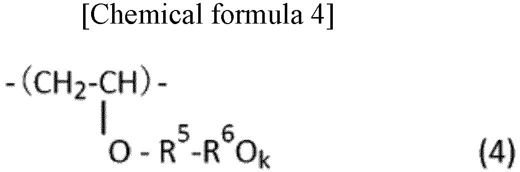

In general formula (4), R⁵ has a structure selected from CH₂ and C₂H₄. R⁶O^{k} is a cyclic ether of any one of a three membered ring and a six membered ring, and the number (k) of the oxygen atom contained in the cyclic ether is k≥1. In the present invention, any hydrogen contained in R⁵ or R⁶ is substituted with at least one of -OH, CH₃ and C₂H₅. That is, the repeating unit of this form has a structure in which a cyclic ether is bonded to the main chain by an ether bond.

In addition, polymer fine particles which are preferably used for drug delivery applications can be obtained by hydrating a biodegradable polymer having a polycarbonate skeleton in the main chain by the hydration method according to the present invention to form fine particles.

Table 1 shows the solubility of PMEA and its analogs in various solvents as examples of the above-mentioned polymers. Each n value in Table 1 means an n value in a polymer having a structure represented by Formula (5) in the side chain portion.

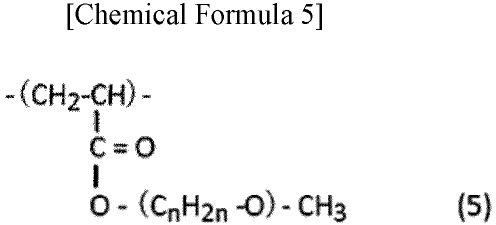

**[Table 1]**

| | | n=2 | n=3 | n=4 | n=5 | n=6 |
|---|---|---|---|---|---|---|
| solvent | water | × | × | × | × | × |
| | ethanol | × | ∘ | ∘ | ∘ | ∘ |
| | methanol | ∘ | ∘ | ∘ | ∘ | × |
| | ethanol: methanol (50:50) | × | ∘ | ∘ | ∘ | ∘ |
| Glass transition temperature | | -35°C | -48°C | -65°C | -78°C | -77°C |
| Contact angle | | 40° | 46° | 51° | 64° | 64° |
| Presence of intermediate water | | present | present | present | present | present |

As shown in Table 1, it is known that the polymer represented by formula (1) is soluble in a specific polar solvent having compatibility with water according to its structure. In addition to the solvents shown in Table 1, various polymers can be dissolved in polar solvents such as DMSO, THF, propanol, acetone, DMF, and acetonitrile, and mixed with water in an indefinite ratio.

(2) A method for hydrating a water-insoluble polymer according to the present invention According to the hydration method according to the present invention, the hydration can be completed in a shorter period of time as compared with a method for hydrating a water-insoluble polymer formed in a film or the like. In addition, by adjusting the conditions for hydrating the polymer dissolved in the polar organic solvent, the hydrated polymer can be precipitated into fine particles, and various functions can be imparted.

According to the present invention, a mechanism by which a water-insoluble polymer can be hydrated in a relatively short time is considered as follows. For example, when the above-mentioned PMEA and the like formed in the form of particles or films are brought into contact with water to be hydrated, since the PMEA and the like are water-insoluble and non-permeable, the process of supplying water molecules to the PMEA molecules present inside the particles is controlled by the speed of the elementary process in which the water molecules contained in the PMEA molecules hydrated near the surface dissociate from the PMEA molecules and rehydrate with other PMEA molecules present in the inner side, and therefore, it is considered that a long time is required to hydrate the whole.

On the other hand, in the present invention, since water molecules are supplied through a predetermined polar solvent in a state where the polymer is dissolved in the polar solvent to cause hydration, the supply of water molecules to the polymer molecules is limited only by the diffusion rate of the water molecules in the polar solvent, and since the diffusion of the water molecules is performed quickly, it is considered that the polymer can be hydrated in a relatively short time.

The method for hydrating a water-insoluble polymer according to the present invention is characterized in that water molecules (aqueous phase) are supplied to a polar solvent in which the polymer is dissolved by an appropriate method to hydrate the polymer in the polar solvent, and then a sufficient amount of the aqueous phase is supplied to substantially replace the solvent in which the polymer hydrate is present with the aqueous phase, thereby causing the solubility of the hydrate of the water-insoluble polymer to be lost and precipitate.

The polar organic solvent used for dissolving the water-insoluble polymer in the present invention is not particularly limited as long as it can dissolve the polymer and has compatibility with water. In particular, methanol, DMSO, THF, propanol, acetone, DMF, acetonitrile and the like are preferably used as solvents in which various polymers have solubility and compatibility with water.

The polar solvent is a good solvent and the aqueous phase is a poor solvent with respect to the water-insoluble polymer handled in the present invention. A means for mixing a solution obtained by dissolving a water-insoluble polymer or the like in a good solvent with a poor solvent, thereby diffusing the good solvent into the poor solvent via an emulsion state to deposit the polymer or the like as dry fine particles is known as a so-called "emulsion solvent diffusion method" or the like as a method for producing spherical solid fine particles.

In the present invention, although the elementary process in which a water-insoluble polymer dissolved in a polar organic solvent is precipitated by mixing with an aqueous phase is not necessarily clear, it is considered that the hydrated water-insoluble polymer is precipitated in a spherical form by passing through an emulsion state or the like according to the surface tension between the polar organic solvent phase and the aqueous phase when the polar organic solvent phase and the aqueous phase are mixed and the mutual diffusion coefficient.

Mixing of a polar organic solvent phase in which a water-insoluble polymer is dissolved with an aqueous phase can be carried out under appropriate conditions. By using a polar organic solvent phase in which a water-insoluble polymer is dissolved, for example, a polar organic solvent phase in which various polymers are dissolved at a ratio of 0.1 to 1 wt%, rapid hydration can be generated when the aqueous phase is mixed, and a fine hydrate can be precipitated. In particular, by using a polar organic solvent phase in which a water-insoluble polymer is dissolved at a ratio of about 0.2 to 0.5 wt%, particles of about 0.1 to 100 µm can be stably formed as precipitates.

On the other hand, when the amount of the water-insoluble polymer dissolved in the polar organic solvent phase is about 0.1 wt% or less, it is difficult for the precipitated polymers to associate with each other, so that particulate precipitates tend not to be obtained. When the amount of the water-insoluble polymer dissolved in the polar organic solvent phase is about 1 wt% or more, the association between the precipitated polymers becomes remarkable and coarse precipitates tend to be formed. When a particulate precipitate is formed using a polar organic solvent phase in which a water-insoluble polymer is dissolved at the concentration, it is preferable to use a component for nucleation or a surfactant component for stabilizing the fine particle shape in order to facilitate the formation of the particulate precipitate.

By preparing, for example, an aqueous phase having a volume equivalent to that of a polar organic solvent phase in which the water-insoluble polymer is dissolved, and pouring the polar organic solvent phase into the aqueous phase, it is possible to obtain a homogeneous liquid phase in which the polymer precipitates in a colloidal state and becomes whitish turbid. Alternatively, a similar whitish homogeneous liquid phase can be obtained by pouring an aqueous phase into the polar organic solvent phase.

The polymer precipitated in the form of particles by the above-mentioned means has a particle of typically about 0.1 to 100 µm as a particle diameter observed under a microscope, and can exist stably for a long period of time in an aqueous phase. Preferably, the precipitated polymer can be used for various purposes by substantially replacing the liquid phase with an aqueous phase by further supplying an aqueous phase to the liquid phase which has become turbid due to the precipitation of the polymer, and then concentrating the precipitated polymer by an appropriate means such as centrifugation.

In addition, in the mixing of a polar organic solvent phase and an aqueous phase in which the above-mentioned polymer is dissolved, an operation such as mixing a small amount of a polar organic solvent phase into the aqueous phase is carried out to cause a rapid substitution between the organic solvent and water, whereby the precipitated polymer can be precipitated on the surface of the liquid phase without being dispersed in the liquid phase as described above, thereby facilitating subsequent recovery and use for various purposes.

On the other hand, mixing a polymer solution in which a polymer is dissolved in a polar organic solvent by bringing an aqueous phase into contact with the solution through a semi-permeable membrane or the like, for example, is also effective in that a time interval for reassembling the polymer molecules precipitated by hydration is secured and the hydrated polymer is stably precipitated as fine particles. At this time, by dissolving a low molecular weight substance having lipophilicity and hydrophobicity in a polar organic solvent, the substance can be supported in the precipitated polymer particles. By utilizing this phenomenon, it is also possible to use a polymer precipitated according to the present invention as fine particles for drug delivery by using a polar organic solvent in which a substance functioning as a drug by exhibiting a predetermined characteristic is dissolved.

The semi-permeable membrane used above is not particularly limited as long as it is a membrane through which a polar organic solvent and water can pass together, and a semi-permeable membrane that can be used as a dialysis membrane, such as a porous membrane of regenerated cellulose, acetyl cellulose, polyacrylonitrile, PTFE, polyester polymer alloy or polysulfone, can be used.

Any polymer which can contain intermediate water by hydration and which is substantially insoluble in the aqueous phase can be precipitated by hydration according to the invention. For example, the polymers described in Table 1 above are water insoluble and can be hydrated by methods according to the present invention. Further, for example, even if the polymer is water-soluble in the state of a homopolymer, it can be hydrated and precipitated according to the present invention by making the polymer substantially insoluble in the water phase by using a copolymer or the like.

(3) Use of a water-insoluble polymer hydrated according to the present invention As described in the above-mentioned Patent Documents 1 and 2, on the surface of a polymer containing intermediate water by hydration, an excellent function as a bio-interface is exhibited by the presence of the intermediate water. Polymers hydrated in accordance with the present invention can also be preferably used to form surfaces that require biocompatibility as is known in the art.

For example, a surface obtained by applying a polymer hydrate hydrated according to the present invention to the surface of a base material can be preferably used as a cell culture support capable of adhering and maintaining cells in a preferred form.

That is, the cell culture support composed of a surface obtained by applying a polymer hydrate hydrated according to the present invention to the surface of a base material can be used without particular limitation as long as it is a cell that lives by adhering to a substrate, and can be applied to the cell culture of epidermal cells, digestive tract epithelial cells such as vascular endothelial cells, oral endothelial cells, esophageal epithelial cells, gastric epithelial cells and intestinal epithelial cells, respiratory epithelial cells such as nasal cavity mucosal epithelial cells, tracheal epithelial cells and alveolar epithelial cells, sweat gland cells, sebaceous gland cells, apocrine gland cells, sebaceous gland cells, apocrine gland cells, mammary cells, etc., endocrine gland cells such as salivary gland epithelial cells, lacrimal gland cells, pancreatic islet cells, adrenal medullary cells, adrenocortical cells, pineal cells, pituitary cells, thyroid cells, etc., endocrine gland cells such as liver cells, renal epithelial cells, pancreatic cells, adrenal cells, etc., visceral parenchymal cells such as hepatocytes, renal epithelial cells, pancreatic cells, adrenal cells, etc., sensory organ cells such as taste bud cells, olfactory epithelial cells, hair cells, etc., nerve cells, glial cells such as astroglia cells, Schwann cells, etc., muscular cells such as cardiac muscle cells, skeletal muscle cells, smooth muscle cells, etc., mesenchymal cells such as fibroblasts, interstitial cells, connective tissue cells, chondrocytes, osteoblasts, etc., thymic epithelial cells, uterine epithelial cells, ovarian follicle cells, fallopian tube epithelial cells, seminiferous tubular epithelial cells, Leydig cells, etc.

Further, a cell culture support comprising a surface obtained by applying a polymer hydrate hydrated according to the present invention to the surface of a base material can be used for culturing various types of stem cells, such as stem cells having pluripotency such as embryonic stem cells (ES cells), induced pluripotent stem cells (iPS cells), embryonic tumor cells (EC cells), embryonic reproductive stem cells (EG cells), nuclear transfer ES cells and somatic cell derived ES cells, tissue stem cells such as hematopoietic stem cells, bone marrow-derived mesenchymal stem cells, adipose tissue-derived mesenchymal stem cells, other interstitial tissue derived stem cells, Muse cells and neural stem cells, tissue stem cells having pluripotency, and progenitor cells in various tissues such as liver, pancreas, adipose tissue, bone tissue and cartilage tissue.

In the culture of stem cells using a cell culture support comprising a surface obtained by applying a hydrated polymer hydrate to the surface of a base material according to the present invention, since the cells can be maintained by adhering in a preferred form, the promotion or suppression of differentiation occurs according to the characteristics of the cultured stem cells, and thus the cell culture can be carried out according to the purpose of the culture.

In addition, when the fine particles composed of a water-insoluble polymer capable of containing intermediate water hydrated by the method according to the present invention were compared with the surface of normal cells such as human skin fibroblasts (NHDFs), selective adsorption such as selective adsorption to highly adhesive cells present in body fluids such as blood was observed. This property can be used for various purposes. For example, as described above, a predetermined drug or the like can be carried in the precipitated polymer fine particles to be used as a carrier for drug delivery targeting a predetermined cell type.

In addition, when polymer fine particles precipitated by the method according to the present invention, particularly fine particles having a particle diameter of several µm or less are used, they are useful as carriers for drug delivery targeting the above-mentioned predetermined cell types in terms of their properties of efficiently incorporating the fine particles into the cells to which they are adhered.

As cells having a high adhesive property present in a body fluid such as the blood, for example, tumor cells such as metastatic cancer cells (CTC) and leukemia cells contained in the blood or the like can be mentioned. It can also be used for the detection of cells such as stem cells, vascular endothelial cells, nerve cells, macrophages, dendritic cells, monocytes, neutrophils, smooth muscle cells, fibroblasts, cardiac muscle cells, skeletal muscle cells, liver parenchymal cells, liver non-parenchymal cells and pancreatic islet cells, and for the detection of other biomarkers.

Hereinafter, a method according to the present invention will be described in more detail using Examples. It should be noted that the following embodiment is one form of the present invention, and the present invention is not limited to this embodiment.

### Example 1

In the following method, PMEA (poly (2-methoxyethyl acrylate)), which is a polymer capable of containing intermediate water by hydration and exhibiting non-water solubility, was dissolved in various polar organic solvents and the solution was mixed with an aqueous phase (purified water), and PMEA dissolved in a polar organic solvent was hydrated and precipitated.

The PMEA used was one synthesized by a known method (for example, see Japanese Patent Application Laid-Open No. 2012-105579). The PMEA was dissolved in methanol, dimethyl sulfoxide (DMSO), and tetrahydrofuran (THF) which are polar organic solvents so as to have a concentration of 0.2 wt%. PMEA was dissolved in both solvents and a clear PMEA solution was formed.

Each PMEA solution (1 mL) was added dropwise to the same volume of purified water at a rate of 0.1 mL/sec using a mechanical pipette (hereinafter, mixing by this method may be referred to as "Oil in Water").). The mixed solution produced by adding each PMEA solution dropwise to purified water was turbid in a colloidal state.

On the other hand, purified water having the same volume as each PMEA solution (1 mL) used was dropped into each PMEA solution at a rate of 0.1 mL/sec using a mechanical pipette (hereinafter, mixing by this method may be referred to as "water in oil").). The mixed solution produced by dropping purified water into each PMEA solution was turbid in a colloidal state.

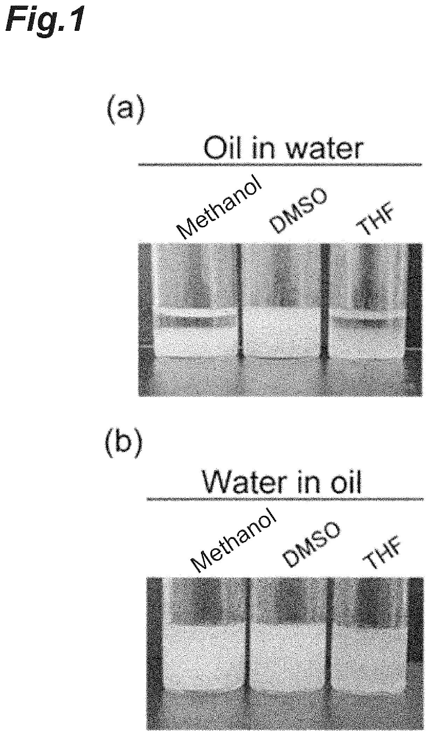

FIG. 1 shows a state of a mixed liquid obtained by mixing the purified water and the PMEA solution. In FIG. 1, (a) shows a PMEA solution mixed with purified water (Oil in Water), and (b) shows a mixed solution when purified water is mixed with PMEA solution (Water in Oil). It can be seen that all of the mixed solutions obtained above were turbid in a colloidal state. The state of the produced colloid tended to change depending on the polar organic solvent used and the mixing method.

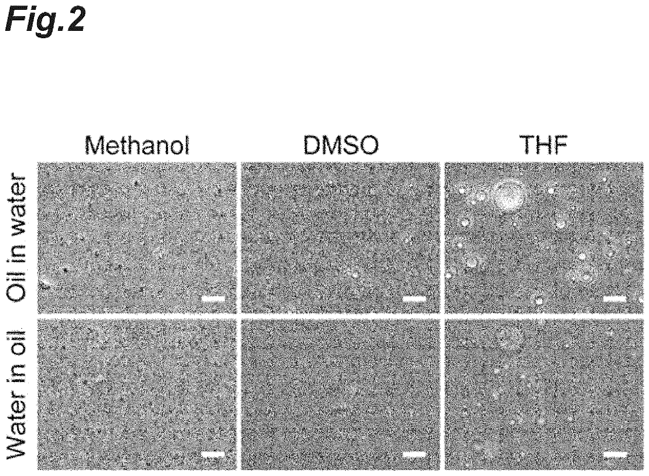

FIG. 2 shows a phase difference microscope image of a turbid (cloud) white aggregate obtained by mixing purified water and a PMEA solution. Each scale bar in FIG. 2 indicates a length of 10 µm. It was confirmed that spherical aggregates with a particle size of about 0.1 to several tens µm were formed regardless of the preparation method. In addition, the average particle diameter and the dispersion state of the particle diameter tended to change due to the difference between the polar organic solvent used and the mixing method.

PMEA solution (DMSO) was added dropwise to purified water (Oil in Water) and allowed to stand at room temperature for a predetermined period of time (10 minutes, 2, 6, 24 hours) in a state of being turbid in a colloidal state, and then centrifuged at 1500 rpm for 3 minutes to precipitate a turbid precipitate and remove the supernatant. Further, the excess water adhered to the precipitate was sufficiently removed with filter paper, and then the precipitate dissolved in deuterated chloroform was subjected to ¹H NMR measurement.

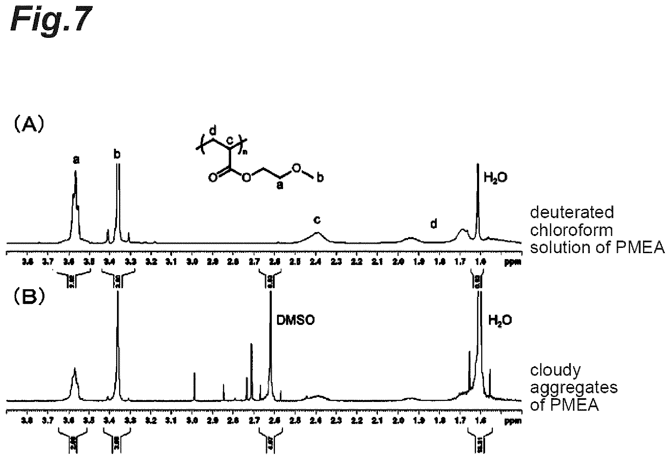

In FIG. 7, as an example of the result of the ¹H NMR measurement, the result of the white turbid aggregate which was allowed to stand for 2 hours after the colloidal purification (B) is shown in comparison with the result of the ¹H NMR measurement of the deuterated chloroform solution of PMEA (A). In the result of ¹H NMR measurement of a deuterated chloroform solution of PMEA (A), peaks (a to d) derived from the structure of each part of PMEA and peaks derived from H₂O mixed by moisture absorption of PMEA are observed. In contrast, in the result of the ¹H NMR measurement of the white turbid aggregate (B), in addition to the peaks (a to d) derived from the PMEA, a peak derived from DMSO remaining in the white turbid aggregate was observed, and it was observed that the peak intensity derived from H₂O became strong.

From the result shown in FIG. 7, it was shown that the white turbid aggregate obtained by Example 1 was mainly composed of PMEA and water molecules, and was considered to be a hydrate in which PMEA was hydrated.

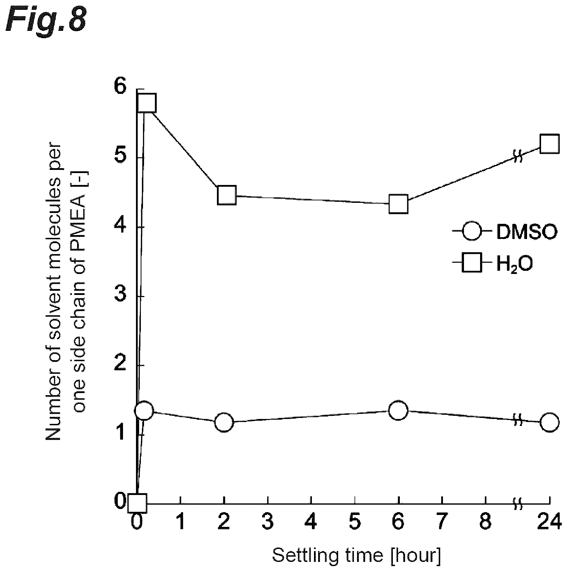

FIG. 8 shows a graph in which the number of molecules of H₂O and DMSO present per one side chain of PMEA calculated from the integrated intensity ratio of the peaks of 3.36 ppm (derived from the methoxy group of PMEA), 1.60 ppm (derived from H₂O) and 2.61 ppm (derived from DMSO) in the result of ¹H NMR measurement of the above-mentioned precipitate is plotted with respect to the retention time as a colloid. As shown in FIG. 8, it was shown that water molecules of about 5 molecules per one side chain of PMEA were contained regardless of the elapsed time after colloid formation, and it was considered that the hydration of PMEA was completed immediately after the mixing of the aqueous phase with the DMSO solution of PMEA.

When the film-like PMEA is saturated with water, the water content is about 9 wt%, while the content of water molecules in the white turbid aggregate is about 40 wt%. This shows that according to the hydration method of the present invention, in addition to being able to perform a hydration treatment on a water-insoluble polymer in a short period of time, it is also able to hydrate a larger proportion of water molecules.

### Example 2

In the following method, PMEA, which is a polymer capable of containing intermediate water by hydration and exhibiting non-water solubility, and polymers which are various analogs thereof, were dissolved in methanol, and a polymer solution was mixed with an aqueous phase (purified water) through a dialysis membrane, and each polymer was hydrated and precipitated. The rate of mixing can be adjusted by mixing the polymer solution and purified water through the dialysis membrane, and precipitates can be substantially recovered in the aqueous phase by using an excess amount of purified water.

The PMEA and PMC3A (poly(3-methoxypropylacrylate), PEEA (poly(2-ethoxyethylacrylate)), PEt2A (poly(2-(2-ethoxyethoxy)ethylacrylate)), PEt2MA (poly(2-(2-ethoxyethoxy)ethylacrylate)), and PTHFA (poly(tetrahydrofurfurylacrylate)) used in the evaluation were each dissolved in methanol at 0.2 wt% and subjected to 0.2 µm filter filtration, and used as polymer solutions.

Dialysis was performed by placing 5 mL of each polymer solution in a dialysis membrane (molecular weight fraction : 3500) made of recycled cellulose and immersing it in a large excess (about 20-fold) of purified water. Distilled water, an external solvent, was exchanged 4 times, and dialysis was performed for 2 days in total. Colloidal cloudiness was observed in the dialysis membrane after dialysis, regardless of which polymer was used.

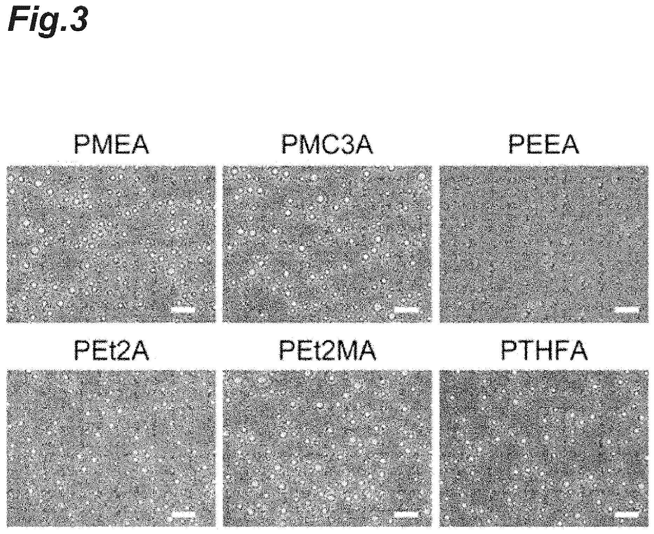

FIG. 3 shows a phase difference microscope image of the white turbid aggregate formed in the dialysis membrane. Each scale bar in FIG. 3 shows a length of 20 µm. For the white turbid aggregate precipitated when the above-mentioned 0.2 wt% PMEA solution was used, a particle size of about 1.2 µm as a Feley diameter by microscope observation, about 0.3 to 1.5 µm as a particle diameter by dynamic light scattering measurement (DLS), and about 5 µm as a particle diameter by volume measurement using a flow cytometer were determined.

In addition, when the white turbid aggregate precipitated using the above-mentioned 0.2 wt% PMEA methanol solution was freeze-dried and dried under reduced pressure to measure the mass of the remaining PMEA polymer, the content of PMEA in the white turbid aggregate was about 1.56 mg/mL, indicating that PMEA was hydrated.

When the colloidal solution in the dialysis membrane after dialysis was diluted 10-100 times each in water, phosphate physiological buffer (PBS), and cell culture medium (containing 10% fetal bovine serum) and kept for 1 week, the spherical shape was not broken and the particle size was maintained. FIG. 3 shows a state in which a colloidal solution in a dialysis membrane after dialysis is diluted in PBS.

### Example 3

The white turbid aggregates containing each polymer obtained in Example 2 were evaluated for their degree of accumulation against human cervical cancer HeLa cells and human normal fibroblasts (NHDFs).

HeLa cells and NHDF were seeded at 1.0 × 10⁵ cells/well in a 24-well plate dish and allowed to stand for 24 hours in an incubator (37°C, 5% CO₂). DMEM/F12 (10% fetal bovine serum, containing penicillin and streptomycin) was used as the culture medium.

The white turbid aggregate containing each of the polymers was added to the HeLa cells and the NHDF cells at a polymer concentration of 150 µg/mL and allowed to stand for 24 hours in an incubator to attempt to adsorb the white turbid aggregate on each cell.

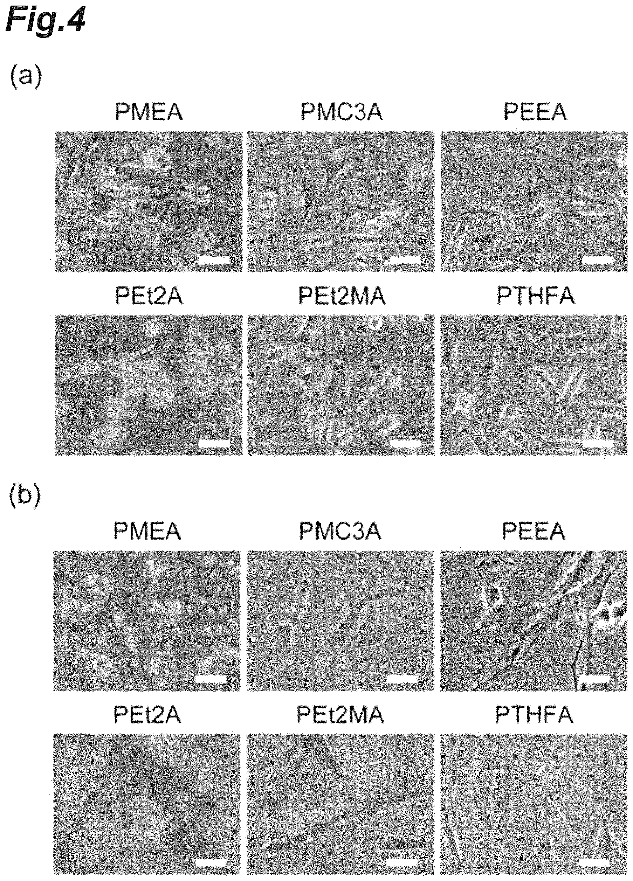

FIG. 4 shows a phase difference microscope image of each cell after the white turbid aggregate is added and allowed to stand. FIG. 4 a) shows HeLa cells, and Figure 4 (b) shows NHDF cells. Each scale bar in FIG. 4 indicates a length of 10 µm.

As shown in Fig. 4, among the polymers evaluated, PMEA, PEt2A, and PEt2MA showed excessive accumulation on HeLa cells, but did not show similar accumulation on NHDF cells. Rather, a distribution avoiding cells was observed. In addition, the white turbid aggregate containing the above-mentioned polymer showed accumulation in human lung cancer cell A549 and human fibrosarcoma HT1080.

In contrast, it was recognized that PMC3A, PTHFA, and PEA accumulated in either HeLa cells or NHDF cells.

From the above results, it is considered that the white turbid aggregate containing PMEA, PEt2A and PEt2MA has cancer cell selective accumulation property. As described in Patent Document 1, it is known that the adsorbable cell species and the like change depending on the amount of intermediate water contained in the polymer and the like. As a result, it is considered that the accumulation in various cells is changed according to the amount of intermediate water contained in the polymer hydrated by the hydration method according to the present invention.

### Example 4

Each of the water-insoluble polymers used in Example 2 is a polymer that exhibits biocompatibility by containing a predetermined amount of intermediate water when hydrated. In contrast, since the white turbid aggregate containing each polymer precipitated by hydration in Example 2 is hydrated in a state of being dissolved in a polar solvent, there is a possibility that the polar solvent may remain in the white turbid aggregate to exhibit toxicity to cells. Therefore, in the following description, the white turbid aggregate containing each polymer obtained in Example 2 was subjected to a hemolysis test and an evaluation of cell viability, and the safety of the white turbid aggregate was evaluated.

### (a) Hemolysis test

Red blood cells (4 × 10⁸ cells) derived from human whole blood (purchased blood) were diluted in Hanks' buffer and seeded in 96-well plate dishes. The white turbid aggregate containing each polymer obtained in Example 2 was diluted in a Hanks buffer solution, mixed with red blood cells derived from human whole blood diluted in the Hanks buffer solution so as to have a concentration of each polymer of about 1000 µg/mL, and allowed to stand at 37 °C for 2 hours.

Thereafter, the 96-well plate dish was centrifuged at 1500 rpm, and the absorbance of the supernatant was measured at a wavelength of 540 nm, and the hemolysis rate (%) indicated by the white turbid aggregate containing each polymer was calculated using the absorbance at the time when red blood cells were treated with 2 % sodium dodecyl sulfate showing high hemolysis as 100 %. Triton X-100, which is used as a positive control for hemolysis, was used for the comparison.

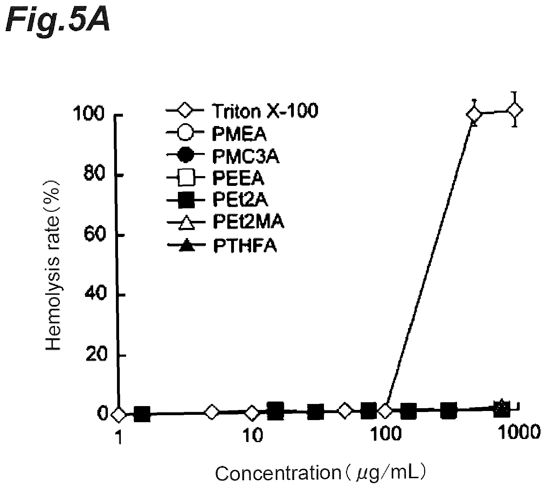

Figure 5A shows the results of the hemolysis test for the white turbid aggregate containing each polymer. As shown in Figure 5A, no hemolysis was observed when the white turbid aggregate containing each polymer used was present at a concentration of 1000 µg/mL.

### (b) Cytotoxicity test

HeLa cells were seeded at 1.0 × 10⁴ cells/cm² in a 96-well plate dish and allowed to stand for 24 hours in an incubator (37°C, 5% CO₂). Next, the white turbid aggregate containing PMEA obtained in Example 2 was added to the HeLa cells so as to have a concentration of each polymer of about 3 mg/mM and allowed to stand for 24 hours in an incubator. The intracellular metabolic activity of HeLa cells in the presence of white turbid aggregates containing each polymer concentration of PMEA was measured using a commercially available cell counting kit-8 (DOJINDO) to evaluate cell viability.

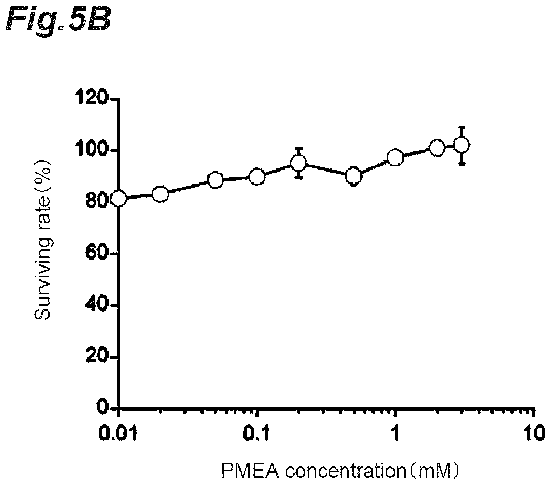

Figure 5B shows the survival of HeLa cells in the presence of white turbid aggregates containing PMEA. As shown in Figure 5B, in the range of PMEA polymer concentration from 0.01 to 3 mg/mM, the survival rate of HeLa cells in the presence of white turbid aggregates containing PMEA did not decrease. Therefore, it was considered that the white turbid aggregates containing PMEA had no cytotoxicity against the cells.

As described above, since hemolysis and cytotoxicity against cells are not observed with regard to the white turbid aggregate formed when the water-insoluble polymer is hydrated in a polar solvent by the hydration method according to the present invention, the concentration of the polar solvent remaining in the white turbid aggregate is considered to be equal to or less than the concentration showing hemolysis and cytotoxicity against cells.

### (c) Evaluation of Surface Active Effect on Cells

In order to evaluate the surface active effect of the white turbid aggregate containing each polymer on cells when the white turbid aggregate is mixed with cells and cultured, HeLa cells after incubation for 24 hours with PMEA, PEt2A and PEEA showing no and PEEA showing no accumulation in Example 3 were washed twice with PBS, the cells were lysed with RIPA buffer, and the total amount of cholesterol in HeLa cells was quantified by the Amplex Red assay. For comparison, untreated HeLa cells (untreated), HeLa cells incubated for 1 hour in a medium containing 10 mM Me-β-CD as a hemolytic agent, HeLa cells incubated for 6 hours in a medium containing 0.77 mM cholesterol, and HeLa cells incubated for 6 hours in a medium mixed with polystyrene beads (pST) were similarly evaluated.

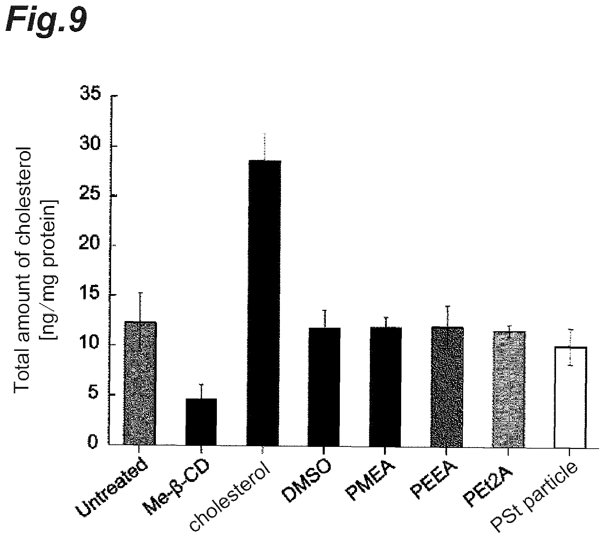

FIG. 9 shows the results of the Amplex Red assay described above. As shown in FIG. 9, in HeLa cells treated with Me-β-CD, cholesterol was eluted by this treatment, and the amount of remaining cholesterol was reduced compared with untreated HeLa cells. On the other hand, in HeLa cells incubated in the presence of PMEA, PEt2A and PEEA hydrated by the method according to the present invention, cholesterol levels similar to those in untreated HeLa cells were observed, and therefore, it was considered that the method did not exhibit a surface active effect on HeLa cells or the like.

(d) Evaluation of Internalization into Cells As shown in Example 3, white turbid aggregates (PMEA hydrates), in which selective accumulation of cancer cells is observed when mixed with cells and cultured, were evaluated by the following method to evaluate how they are taken up by cells and internalized during the culture.

A fluorescence-labeled PMEA was synthesized by modifying the thiol end of a PMEA (number average molecular weight: 22000) polymerized by reversible addition-cleavage chain transfer polymerization (RAFT) using 2-(Dodecylthiocarbonothioylthio)-2-methylpropionicacid with a fluorescent group (Bodipy), and a white turbid aggregate obtained by hydrating a fluorescent PMEA (EF-PMEA) obtained by mixing 5 wt% of the fluorescence-labeled PMEA with the unmodified PMEA according to the method described in Example 2 was added to HeLa cells in the same manner as in Example 3, and the mixture was allowed to stand in an incubator for culture, except that the white turbid aggregate was used.

Cells cultured for 24 hours were washed twice with PBS, and then subcellular organelles, lysosomes, endoplasmic reticulum and mitochondria, were stained with Lyso Tracker, ER-Tracker and Mito Tracker, respectively, and the localization of fluorescent PMEA to subcellular organelles was assessed by confocal laser microscopy. As an index of colocalization rate, correlation coefficient between fluorescent PMEA and Pearson of each organelle was calculated by image analysis using image J.

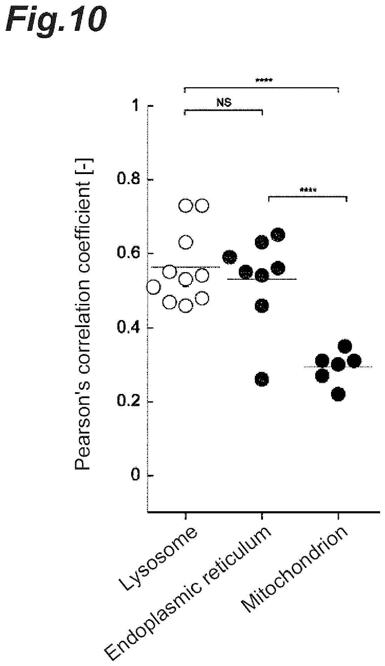

FIG. 10 shows the correlation coefficient with fluorescent PMEA in lysosome, endoplasmic reticulum and mitochondrion calculated above. As shown in FIG. 10, fluorescent PMEA was observed to show a high correlation coefficient with lysosomes and endoplasmic reticulum. These results suggest that fluorescent PMEA enters the cell through endocytic pathway. Furthermore, the high correlation coefficient of fluorescent PMEA with the endoplasmic reticulum indicates that fluorescent PMEA entering the lysosome was further transferred to the endoplasmic reticulum by the route through the endosome escape.

When a drug is directly delivered to a small organ in the cytoplasm by the so-called Drug Delivery System (DDS), it is most likely that the drug enters the cell through the cell membrane through endocytosis, and then it is necessary to transfer the drug to another organelle such as the endoplasmic reticulum. In the turbid aggregate produced by hydrating fluorescent PMEA by the hydration method according to the present invention, as described above, the entry into cells through endocytosis and the subsequent migration into the endoplasmic reticulum or the like suggest that the turbid aggregate functions effectively as a carrier for DDS, and it is expected that the same produced in the non-fluorescent PMEA or the like.

When DDS is performed starting from intravenous administration or the like, it is necessary to suppress the interaction between the DDS carrier and blood, especially to prevent platelets and the like from being adsorbed and aggregated on the DDS carrier, and it is necessary to impart bioaffinity (blood compatibility) to the surface of the DDS carrier. As a specific means for imparting biocompatibility to the surface of a DDS carrier, a technique of coating the surface of the DDS carrier with PEG having biocompatibility has been studied.

In the method described below, the rate of cell internalization of fluorescent PMEA hydrated by the hydration method according to the present invention described above was evaluated in comparison with PEG.

Fluorescent PEG (EF-PEG) in which one end of PEG having Mn of 20000 was fluorescently labeled with a fluorescent group (Bodipy) so that the number average molecular weight (Mn) was similar to the fluorescent PMEA was used as a comparison. In addition to untreated NHDFs and HeLa cells, we used HeLa cells pretreated for one hour with culture medium (DMEM/F12 (10% fetal bovine serum, containing penicillin and streptomycin)) supplemented with Heparinase (2U), which degrades heparin, Hyaluronidase (10U), which cleaves hyaluronic acid, and Neuraminidase (0.1U), which cleaves polysialic acid, for the purpose of assessing the effects of cell surface carbohydrate chains.

Each of the cells was seeded in a culture medium (DMEM/F12 (10% fetal bovine serum, containing penicillin and streptomycin)) using a 12-well plate dish, and white turbid aggregate (150 µg/mL) prepared using fluorescent PMEA as described above or the fluorescent PEG was added to each cell, and the cell was allowed to stand in an incubator for 3 hours. At this time, the number of fluorescent molecules contained in the fluorescent PEG was made equal to the number of fluorescent molecules contained in the fluorescent PMEA, so that the absorbance was made equal. Each of the stationary cells was washed twice with PBS, the cells were removed from the dish by trypsin treatment, and the integral value of fluorescence intensity per cell was measured by a flow cytometer.

Table 2 shows the relative values of the average fluorescence intensity measured above. As shown in Table 2, when EF-PEG was mixed, there was no substantial change in fluorescence intensity after culture compared with the case where no fluorescence component was mixed in either HeLa cells or NHDF cells (untreated), while an increase in fluorescence intensity was observed when EF-PMEA was mixed and cultured. These results suggest that EF-PMEA is more rapidly taken up by cells than EF-PEG.

When EF-PMEA was mixed and cultured, the increase in fluorescence intensity was greater in HeLa cells than in NHDF cells, probably due to the selective adsorption of EF-PMEA on HeLa cells. The difference in fluorescence intensity caused by the change in the sugar chain of the cell membrane of HeLa cells suggests that the intracellular uptake of EF-PMEA is related to the structure of the cell surface.

**[Table 2]**

| | Pretreatment | EF-PMEA | EF-PEG | ^{∗}Untreated |
|---|---|---|---|---|
| Hela | - | 45.0±1.47 | 0.62±0.09 | 0.37±0.03 |
| | Hep. | 41.9±2.69 | 0.47±0.04 | |
| | Hya. | 22.7±5.41 | 0.44±0.01 | |
| | Neu. | 87.0±8.19 | 0.48±0.06 | |
| NHDF | - | 2.7±1.47 | 0.57±0.02 | 0.50±0.02 |

(e) Evaluation of Interaction between White Turbid Aggregates and Sugar Chains According to the Invention As described above, it was observed that the degree of incorporation into cells of the white turbid aggregates obtained by hydrating the above-mentioned fluorescent PMEA by the hydration method according to the invention changes according to the state of the sugar chains in the cell membrane.

Using Hyaluromic acid (weight-average molecular weight: 30000) and dextran-70 (weight-average molecular weight: 70000) as sugar chains, DMT-MM and Carbonyldiimidazole (CDI), respectively, fluorescence was induced by condensation reaction with fluorescent molecules having amino groups (EF - Hyaluromic acid and EF-dextran), and each 1 mg was dissolved in 900 µL of PBS. The solution was mixed with a white turbid aggregate (polymer weight: 100 mg) obtained by hydrating PMEA according to the method described in Example 2, allowed to stand for 6 hours, and the localization of fluorescent molecules was evaluated by a fluorescence microscope.

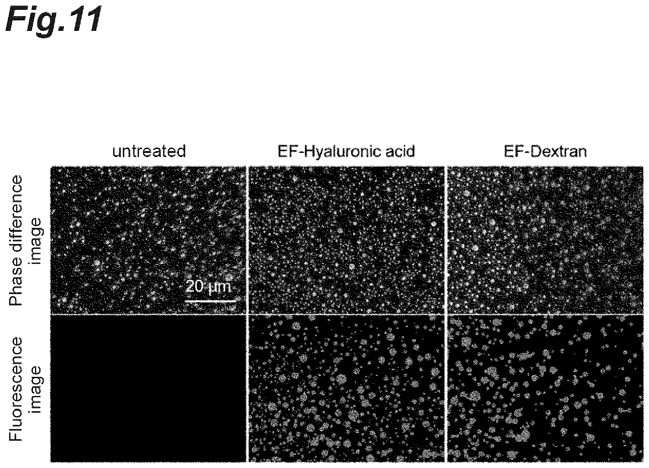

FIG. 11 shows a phase difference image and a fluorescence image of the white turbid aggregate mixed with the fluorescent sugar chains in comparison with those of the white turbid aggregate not mixed with sugar chains. As shown in FIG. 11, each sugar chain was observed to exist by being adsorbed on a turbid aggregate, indicating that affinity exists between the turbid aggregate and the sugar chain.

### Example 5

In this example, an attempt was made to carry a substance exhibiting a predetermined function as a drug in a turbid white aggregate produced when hydrated by the hydration method according to the present invention.

A polymer solution was prepared by dissolving 2 mg of PMEA and 0.5 mg of doxorubicin (DOX), which is a fat-soluble anticancer drug, in 1 mL of methanol, and placed in a dialysis membrane (molecular weight fraction : 3500) made of regenerated cellulose in the same manner as in Example 2, followed by dialysis against a large excess (about 20 times) of purified water. The dialysis was carried out for 3 days while changing the purified water until the coloring of the external solvent by the elution of DOX was sufficiently removed. Colloidal cloudiness was observed in the dialysis membrane after dialysis, and turbid aggregates were formed.

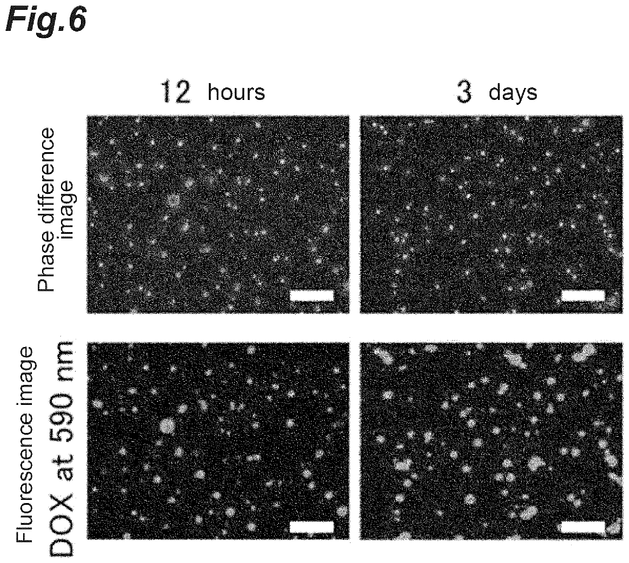

FIG. 6 shows the result of observing the white turbid aggregate obtained by a fluorescence microscope. Each scale bar in FIG. 6 indicates a length of 10 µm. As shown in FIG. 6, in fluorescence microscopy (after 12 hours) of the white turbid aggregate obtained, a red fluorescence of doxorubicin (excitation wavelength 480 nm, fluorescence wavelength 590 nm) was observed only at the same position as that of the spherical aggregate, indicating that doxorubicin was incorporated into the aggregate. In addition, the white turbid aggregate was diluted in PBS, allowed to stand in an incubator at 37 ° C, and then the doxorubicin content was confirmed by fluorescence microscopy (after 3 days).

An excessive amount of methanol was added to the turbid white aggregate after 3 days had passed since the above formation to dissolve it, and the amount of doxorubicin present was determined by measuring the absorbance (480 nm), and the efficiency of the incorporation of doxorubicin into the turbid white aggregate was calculated from the ratio to the amount of doxorubicin used in the above hydration. As a result, the entrapment efficiency of doxorubicin in the white turbid aggregate containing PMEA was 26.4%.

The encapsulation of doxorubicin is considered to be a result of taking in fat-soluble doxorubicin when PMEA dissolved in a good solvent is replaced with purified water by dialysis and hydrated to precipitate. Similar internalization was observed with doxorubicin as well as with the anticancer agents mitoxantrone, the fluorescent molecules fluorescein, rhodamine, 6-p-toluidinyl naphthalene-2-sulfonate, and the fluorescent lipid molecule laurdan.

From the above, it is considered that the aggregate precipitated in the hydration method according to the present invention can contain a wide range of fat-soluble (hydrophobic) drugs, physiologically active substances, lipids and synthetic compounds, and can be used as a carrier for drugs having anti-cancer properties and the like, or for separation of physiologically active substances in the vicinity of cancer tissues, together with selective accumulation in cancer cells and the like due to the presence of intermediate water.

### Industrial applicability

The water-insoluble polymer hydrated by the method according to the present invention can be preferably used for forming a surface where biocompatibility is required. The selective adhesion to cells can be used for detection of various types of cells.

## Claims

1. A method for hydrating a water-insoluble polymer capable of containing intermediate water, comprising;
a solution generation step of dissolving a water-insoluble polymer capable of containing intermediate water by hydration in a polar organic solvent to obtain a solution, and
a precipitation step hydrating and precipitating the water-insoluble polymer by mixing the solution with an aqueous phase.

2. The method for hydrating a water-insoluble polymer capable of containing intermediate water according to claim 1, wherein the water-insoluble polymer capable of containing intermediate water by hydration is a polymer having a side chain portion containing a chain ether structure or a cyclic ether structure with respect to at least a part of the main chain skeleton.

3. The method for hydrating a water-insoluble polymer containing intermediate water according to claim 1 or 2, wherein at least a part of the water-insoluble polymer is precipitated in a colloidal state in the precipitation step.

4. The method for hydrating a water-insoluble polymer capable of containing intermediate water according to any one of claims 1 to 3, wherein in the precipitation step, the solution and the aqueous phase are mixed via a semi-permeable membrane.

5. The method for hydrating a water-insoluble polymer capable of containing intermediate water according to any one of claims 1 to 4, wherein a fat-soluble drug is further dissolved in the solution.

6. A composition containing hydrated water-insoluble polymer molecules, in which a water-insoluble polymer is hydrated and precipitated by mixing an aqueous phase with a solution obtained by dissolving water-insoluble polymer capable of containing intermediate water by hydration in a polar organic solvent.

7. The composition containing the hydrated water-insoluble polymer molecule according to claim 6, having a particle shape.

8. The composition containing the hydrated water-insoluble polymer molecule according to claim 6 or 7, having 100 µm or less of an average particle diameter.
